# EUROPEAN PATENT APPLICATION

(11) **EP 3 001 197 A1**
(43) Date of publication of application: **30.03.2016**
(21) Application number: 14003291.3
(22) Date of filing: 23.09.2014
(51) Int. Cl.: G01N 33/533, G01N 1/30, G01N 33/58

(54) **Fluorescent protein stains**

(71) Applicant: Universität Heidelberg, 69117 Heidelberg (DE)
(72) Inventor: Klein, Christian, 69214 Eppelheim (DE); Jöst, Christian, 69120 Heidelberg (DE)
(74) Representative: Müller-Boré & Partner Patentanwälte PartG mbB

(57) **Abstract**

The present invention relates to novel staining reagents and compositions for biomolecules as well as methods of using and preparing the same.

## Description

The present invention relates to novel staining reagents and compositions for biomolecules as well as methods of using and preparing the same.

Fluorescent dyes are widely used for staining, detecting, and/or quantifying biomolecules in a sample. In general, for many applications that utilize fluorescent dyes as detection tools, it is necessary to conjugate the fluorescent dye with a ligand such as a protein, peptide, nucleotide, nucleic acid, and other biomolecules to make a dye-labeled ligand. Therefore, a reactive linker moiety is needed, which is ideally sufficiently reactive towards a large number of biomolecules while also being reasonably stable in solution without rapid degradation.

The detection and analysis of proteins is of particular interest to the biological community. Primarily, proteins are detected and characterized using gel electrophoresis. Among various variants of gel electrophoresis, a technique called sodium dodecylsulfate polyacrylamide gel electrophoresis (SDS-PAGE) is prominent and widely used due to its capability to separate proteins by their size rather than their size-to-charge ratio, which facilitates the interpretation of resulting protein bands. The necessary subsequent staining step is often carried out using one of the two widely used stains known as Coomassie brilliant blue (Chemical Abstract Service (CAS) Registry Number 6104-59-2) or silver staining. However, staining protocols involving either of these two staining methods are very time consuming (approx. 4 hours) and require multiple working steps. Moreover, detection methods employing Coomassie Brilliant Blue tend to only allow for detection limits in the range of 10 ng of protein. Similar limitations are known for the covalent staining of other biomolecules, such as deoxyribonucleic acids (DNA).

Thus, there is still a need for staining reagents for biomolecules, such as proteins or DNA, which afford reliable and sensitive detection and which require little time and few working steps.

Accordingly, the technical problem underlying the present invention is to provide novel staining reagents and compositions for biomolecules, in particular proteins, but also DNA, RNA; lipids, sugars and low molecular metabolites, as well as methods of using and preparing the same.

In a series of experiments the applicant has found that fluorescent dyes bearing a bromoacetyl sidechain as a reactive linker moiety are excellent in labeling biomolecules, in particular proteins, due to their highly promiscuous binding characteristics. At the same time, staining reagents comprising the bromoacetyl sidechain are stable in aqueous and organic solutions. The bromoacetyl sidechain is reactive towards biomolecules having nucleophilic functional groups, especially thiol-, amino-, imino-, alcohol-or carboxyl-containing functional groups, particularly thiol-containing functional groups. These functional groups can be present in organic molecules of all types, including biochemical metabolites, pharmaceutical agents, diagnostic agents, agrochemicals, molecular probes, and all type of biomolecules. A particular useful application described in more detail hereinbelow, stems from the reactivity towards proteinaceous compounds.

Thus, in the course of the present invention, there has been developed a novel, exceptionally fast and sensitive SDS-PAGE-stain that does not require any additional staining steps after the gel run, thus lowering the total time in which results are obtained to less than 1 hour. This is a time saving of ~4 hours compared to the two most widely used stains (Coomassie and silver stain). If a fluorescent readout is used, the sensitivity is equal to the most sensitive protein stains currently available.

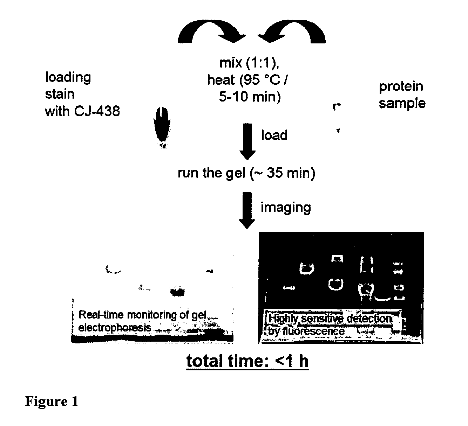

The staining is performed during the sample preparation with a ready-to-use formulation ("loading stain"). The ensuing workflow (cf. Figure 1) is identical to the standard procedure for SDS-PAGE, with the significant difference that no additional staining after the electrophoretic separation is needed. Amounts of ∼1 µg protein can be detected by visual inspection without any special equipment. When using a fluorescence-imager, the sensitivity is comparable to the tedious silver staining method and low nanogram amounts of protein can be detected.

Thus, in a first aspect, the present invention provides a staining reagent for biomolecules according to the general formula (1): 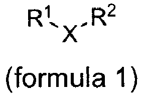
wherein R¹ is a fluorescent dye selected from the group consisting of Rhodamine B, Rhodamine 6G, Rhodamine 123, Sulforhodamine B, Sulforhodamine 101, Carboxy-Rhodamine, Rosamine, Texas Red, Fluorescein, Carboxyfluorescein, Cy3, Cy5, Cy7, Nile blue, quinine and the class of BODIPY dyes, and
wherein R² is a reactive moiety according to the following formula (2) 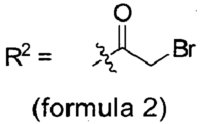 and wherein X is a spacer or X is absent, such that R¹ and R² are connected via a single bond.

According to the present invention, the term "biomolecule" refers to peptides, proteins (including enzymes and antibodies), deoxyribonucleic acids (DNA) and ribonucleic acids (RNA).

According to the present invention,
Rhodamine B is [9-(2-carboxyphenyl)-6-diethylamino-3-xanthenylidene]-diethylammonium chloride (CAS number 81-88-9),
Rhodamine 6G is [9-(2-ethoxycarbonylphenyl)-6-(ethylamino)-2,7-dimethylxanthen-3-ylidene]-ethylazanium (CAS number 989-38-8),
Rhodamine 123 is 6-amino-9-(2-methoxycarbonylphenyl)xanthen-3-ylidene]azanium chloride (CAS number 62669-70-9),
Sulforhodamine B is 2-(3-diethylamino-6-diethylazaniumylidene-xanthen-9-yl)-5-sulfo-benzenesulfonate (CAS number 3520-42-1),
Sulforhodamine 101 is 9-(2,4-Disulfophenyl)-2,3,6,7,12,13,16,17-octahydro-1H,5H,11H,15H-xantheno[2,3,4-ij:5,6,7 -i'j']diquinolizin-18-ium inner salt (CAS number 60311-02-6),
Texas Red is Sulforhodamine acid chloride (CAS number 82354-19-6),
Fluorescein is 3',6'-dihydroxyspiro[2-benzofuran-3,9'-xanthene]-1-one (CAS number 2321-07-5), Carboxyfluorescein is 3',6'-dihydroxy-3-oxospiro[2-benzofuran-1,9'-xanthene]-6-carboxylic acid (CAS number 3301-79-9),
Cy3 is 2-[(E,3Z)-3-(1,3-dihydroindol-2-ylidene)prop-1-enyl]-3H-indol-1-ium,
Cy5 is 2-[(1E,3E,5Z)-5-(1,3-dihydroindol-2-ylidene)penta-1,3-dienyl]-3H-indol-1-ium,
Cy7 is 1-(5-carboxypentyl)-2-[7-(1-ethyl-5-sulfo-1,3-dihydro-2H-indol-2-ylidene)hepta-1,3,5-trien-1-yl]-3H-indoli-um-5-sulfonate,
Nile blue is [9-(diethylamino)benzo[a]phenoxazin-5-ylidene]azanium;sulfate (CAS number 3625-57-8).
The class of BODIPY dyes is based on the common structural element 4,4-difluoro-4-bora-3a,4a-diaza-s-indacene.

According to the present invention, the term "spacer" refers to a chemical structural element whose purpose is to spatially separate two functional parts in the staining reagent, i.e. R¹ and R². Typical examples for spacers include aliphatic hydrocarbons or aromatic rings.

According to a preferred embodiment, if X is not absent, X is a spacer according to the general formula (3) 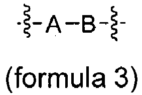 wherein
A is an aliphatic hydrocarbon with 1 to 10 carbon atoms and optionally having (i) two terminal hydroxyl groups, (ii) two terminal amino groups, (iii) two terminal methylamino groups, (iv) one terminal hydroxy group and one terminal amino group or (v) one terminal hydroxy group and one terminal methylamino group, each of the groups being at the opposite terminals of the aliphatic hydrocarbon group, and wherein A is bound to R¹ via an ester or an amide bond and wherein A is bound to B or R² via an ester or an amine bond, and wherein
B is aminobenzoic acid, which is bound to R¹ or A via an ester or an amide bond and which is bound to R² via an ester or an amide bond with the proviso that, if X is not absent, at least one of A and B is present.

According to a preferred embodiment, the staining reagent for biomolecules is a staining reagent for biomolecules selected from any one of the following formulae (4) to (9):

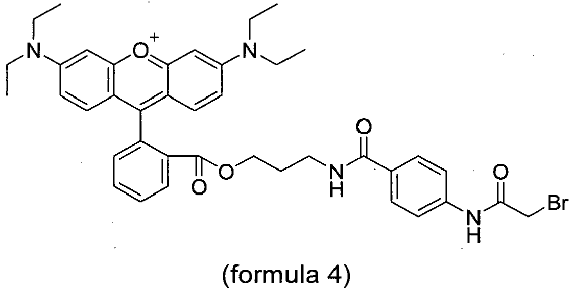

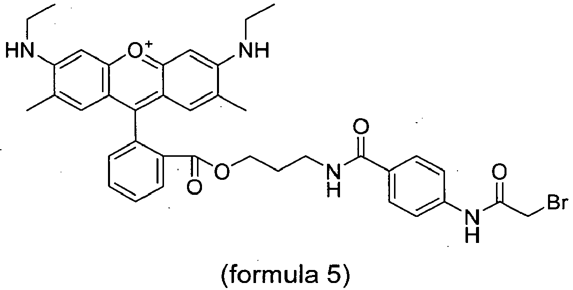

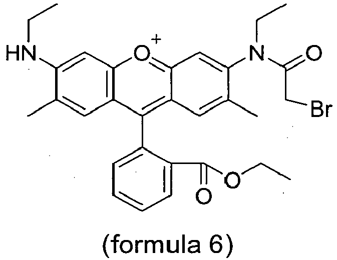

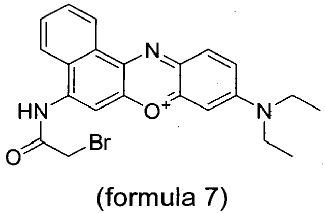

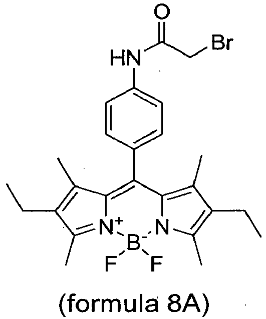

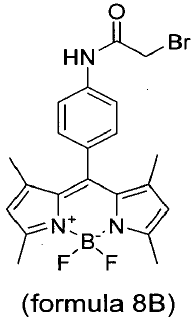

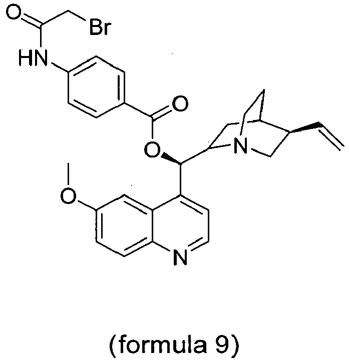

The respective counterions in the above formulae can be any one of the commonly known ones regarding such compounds like for example CH₃COO⁻, CF₃COO⁻, halogenide anions, sulfate, phosphate, etc.

A second aspect of the present invention relates to a method of staining a protein sample with the staining reagent for biomolecules, wherein the staining reagent and a protein sample comprising one or more proteins are reacted and conjugates of said protein staining reagent with one or more proteins are produced. In the same way, also a DNA sample can be stained.

According to the present invention the term "reacted" refers to the formation of a single bond between the protein and the staining reagent. In particular, said reaction involves a nucleophilic attack of a thiol, amine or carboxylic acid of the protein at the carbon atom with a neighboring bromide atom.

According to the present invention, the proteins are reacted with the staining reagent for biomolecules by mixing the protein sample and a staining reagent composition at a ratio of 0.1:1 to 1:0.1 (V/V), wherein a range of 0.5 to 1 to 1: to 0:5 (V/V) is preferable and 1:1 (V/V) is especially preferable. The staining reagent composition comprises the staining reagent which is dissolved in an aqueous solution. The staining reagent may be present in the staining reagent composition in concentrations (prior to mixing) from 0.1 mM to 50 mM, wherein 0.5 mM to 20 mM is preferable, 1 mM to 10 mM is more preferable and 1 mM to 2 mM is most preferable.

Furthermore, a buffer reagent may be present in the staining reagent composition. While the identity and concentration of the buffer reagent is not strictly limited, the buffer reagent should be capable of maintaining a pH range of 7.0 to 9.5 and more preferable a pH range of 7.5 to 8.8 and most preferably a pH range of 7.8 to 8.3. Lower pH values result in lowered sensitivity and broadened bands in conjunction with subsequent protein separation by gel electrophoresis whereas higher pH values results in broader and unsymmetrical bands in conjunction with subsequent protein separation by gel electrophoresis. The person skilled in the art readily knows, which buffer reagents and concentrations may fit such needs. In particular, sodium or potassium phosphate buffers, tris(hydroxymethyl)aminomethane hydrochloride (Tris-HCl) buffers, 3-(N-morpholino)propanesulfonic buffers (MOPS), 2-(N-morpholino)ethanesulfonic buffers (MES) and 4-(2-hydroxyethyl)-1-piperazineethanesulfonic buffers (HEPES), all purchasable from commercial suppliers, are suitable at concentrations ranging from 50 mM to 400 mM and preferably from 100 mM to 200 mM.

Furthermore, the staining reagent composition may contain sodium dodecylsulfate in a concentration range of 1% to 10% (V/V) and preferably 2% to 4% (V/V) in order to mask the inherent charges of the proteins in the protein sample upon mixing with the protein staining reagent composition. If less sodium dodecylsulfate is used, not all charges of the protein may be masked.

Moreover, glycerol may be present from 2% to 40% (VN) and preferably from 10% to 20% (V/V).

All necessary chemicals mentioned herein are readily commercially available.

After mixing, the temperature is increased to 80 °C to 100 °C, preferably 90 °C to 97 °C and more preferably 95 °C for a fixed time period (see paragraph below). If the temperature is too low, the reaction is not complete. At temperatures higher than 97 °C the aqueous solution starts to boil and therefore the relative composition may be subject to unwanted changes.

The reaction time at elevated temperature is preferably from 1 to 120 min, more preferably from 3 to 10 min and most preferably from 5 to 10 min. If the reaction time is less than 3 min, then the fraction of unreacted proteins may be high. If the reaction time is higher than 10 min, the time consumption of the procedure is unnecessarily increased.

According to a preferred embodiment of the method, a protein sample comprising one or more proteins is subjected to a protein separation procedure prior to being reacted with the staining reagent for biomolecules.

According to the present invention, protein separation procedures include gel electrophoresis, e.g. polyacrylamide gel electrophoresis (with or without sodium dodecylsulfate), affinity chromatography (e.g. immobilized metal affinity chromatogaphy, IMAC) and size exclusion chromatography.

According to another preferred embodiment of the method, a protein sample comprising one or more proteins is subjected to a protein separation procedure after being reacted with the staining reagent for biomolecules.

According to a preferred embodiment of the method, the reacted protein sample is monitored during separation using a fluorescence detection method.

According to the present invention, monitoring may be performed using an ultra-violet light source directed at the gel in order to observe the fluorescence of the conjugate.

According to another preferred embodiment of the method, the protein sample comprising one or more proteins is subjected to polyacrylamide gel electrophoresis and the gel is not washed after performing the reaction and prior to fluorescent detection of the protein bands.

According to the present invention, washing steps that are typically performed for conventional stains, such as Coomassie Brilliant Blue, in order to reduce background staining, can be omitted. In particular, conventional washing steps include immersing the resulting gel into baths of methanol and acetic acid or water.

A third aspect of the present invention relates to a method for producing the staining reagent for biomolecules, wherein a fluorescent dye selected from the group consisting of Rhodamine B, Rhodamine 6G, Rhodamine 123, Sulforhodamine B, Sulforhodamine 101, Carboxy-Rhodamine, Rosamine, Texas Red, Fluorescein, Carboxyfluorescein, Cy3, Cy5, Cy7, Nile blue, quinine and BODIPY is reacted with the reactive moiety R² either directly, if X is absent, or via the spacer X. Examples for such a method are given in the section titled Examples.

It is a fourth aspect of the present invention to provide a kit comprising a stock solution of the staining reagent for biomolecules.

A kit according to the present invention refers to a container which comprises reagents in smaller separate containers packaged. The kit contains a stock solution of the staining reagent and may further comprise other chemicals, such as buffering agents, antioxidants, surfactants, organic solvents or additional detection reagents in the same or different solutions. One or more components of the kit are mixed to create a ready-to-use solution of the staining reagent for biomolecules.

A buffer reagent according to the present invention is a buffer salt in an aqueous solution, which is suitable to maintain a pH range of 7.0 to 9.5 and more preferable a pH range of 7.5 to 8.8 and most preferably a pH range of 7.8 to 8.3 in a 1:1 (V/V) mixture of the ready-to-use staining reagent for biomolecules and a sample containing one or more proteins. Examples of buffer salts include sodium or potassium phosphate buffers or Tris-HCl, which is commercially available.

A fifth aspect of the present invention relates to the use of the staining reagent for biomolecules for staining a protein sample comprising one or more proteins.

A sixth aspect of the present invention relates to the use of the staining reagent for biomolecules for staining a tissue for microscopic analysis.

A tissue according to the present invention relates to any biological tissue slice, wherein the thickness is not particularly limited.

A seventh aspect of the present invention relates to a prestained protein molecular weight marker composition, comprising at least two different proteins, said at least two proteins having differing molecular weights, wherein said at least two proteins are stained with at least one staining reagent according to any one of claims 1 to 3.

Protein molecular weight marker compositions according to this aspect are not particularly limited and are known in the art. Exemplary proteins that can be used in such compositions include β-galactosidase from *E. coli,* bovine serum albumin, methionine aminopeptidase from *E. coli,* and lysozyme from chicken egg-white.

The different proteins in the prestained protein molecular weight marker composition of the present invention can all be stained with the same staining reagent of the present invention. Alternatively, each of said different proteins can be stained with a different staining reagent of the present invention. Suitable buffers in which the compositions can be stored are not particularly limited and are known in the art.

An eighth aspect of the present invention relates to a method for producing the prestained protein molecular weight marker composition of the present invention, comprising the steps of
(a) providing said at least two proteins in a buffer that is free of thiols and
(b) reacting said at least two proteins with at least one staining reagent according to the present invention, thereby producing conjugates of said at least one staining reagent with said at least two proteins.

The above method can be performed batch-wise, i.e., by providing a buffer that is free of thiols, said buffer comprising all of the at least two proteins, and reacting all of the at least two proteins with a staining reagent of the present invention. This results in a prestained protein molecular weight marker composition wherein all of the different proteins are stained with the same staining reagent. Alternatively, the above method can be performed separately for each of the at least two proteins, *i.e.,* by providing said proteins individually in a buffer that is free of thiols, staining each of the individual proteins with a different staining reagent of the present invention, and combining the at least two different proteins. This results in a prestained protein molecular weight marker composition wherein all of the different proteins are stained with a different staining reagent.

The Figures show:
Figure 1 shows: Schematic representation of an exemplary work flow. In particular, the specified parameters are not limiting the scope of the present invention.
Figure 2 shows: Staining of a SDS-PAGE gel after separation of a test protein set using the staining reagent according to formula 4 (upper row, A) and using Coomassie Brilliant Blue (bottom row, B).
Figure 3 shows: Dynamic range and detection limit for staining proteins using the staining reagent according to the formula 4.
Figure 4 shows: Fluorescence intensity of different protein dilutions (2-200 ng). A: Fluorescence image, B: Silver staining image. C: The 5 plots represent the fluorescence intensity in the CJ-438 stained image (A). 1: β-galactosidase (120 kDa), 2: bovine serum albumin (66 kDa), 3: ovalbumin (45 kDa), 4: lactate dehydrogenase from porcine muscle (35 kDa), 5: Bsp 981 from *E.coli (25 kDa).*
Figure 5 shows: An interference gel test. Protein in each pocket: bovine serum albumin (30 µg). Lane 1: 1 M acetic acid, 2: 1 M ammonia, 3: 8M urea, 4: 2M thiourea, 5: 1 M Tris, 6: 1 M MOPS.

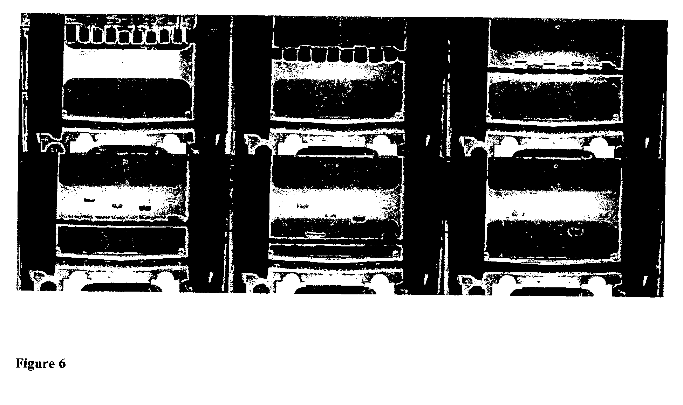

Figure 6 shows: Real-time monitoring of an SDS-PAGE experiment using a staining reagent for biomolecules according to the present invention. A: sample loading; runtime: B: 6 min, C: 12 min, D: 18 min, E: 24 min, F: 35 min.
Figure 7 shows: Rhodamine B and 6G based stains. Upper band: BSA (66 kDa) stained with CJ-462, lower band: *E. coli* methionine aminopeptidase (35 kDa) stained with CJ-438. Both proteins were stained separately, the stains quenched with DTT and both proteins combined.
Figure 8 shows: Lanes 1-3: different amounts of a protein marker produced by a method according to the present invention. Band I: β-galactosidase from *E. coli* (apparent size 108 kDa); II: bovine serum albumin (apparent size: 59 kDa); III: Methionine aminopeptidase from *E. coli* (apparent size 32 kDa); IV: lysozyme from chicken egg-white (not separated from the running front)
   Lane 4: fluorescent protein marker produced by a method according to the present invention, 5.6 µg protein per band

The staining reagents for biomolecules of the present invention advantageously overcome a variety of drawbacks known from other, conventional staining reagents. In particular, staining reagents according to the present invention are faster and easier to use. For instance, when used in conjunction with SDS-PAGE, washing or destaining steps can be omitted without a loss in sensitivity of the detection method. When running an SDS-PAGE experiment, colored gel bands can be observed already after approx. 50 min including sample preparation, electrophoretic separation and imaging. Furthermore, it is possible to observe first bands separating from the running front within 15 min, enabling ultra-fast evaluation of protein purification steps like histidine-tag IMAC or size exclusion columns. Moreover, the sensitivity and dynamic range are with 1 ng and >5000, respectively, excellent and exceed most other, established methods. The staining reagents for biomolecules according to the present invention modify a range of protein residues making them superior to most other stains because the absence of one particular amino acid in a protein can be tolerated. In contrast to those stains that target exclusively lysine residues and which require tedious fine-tuning of staining conditions to achieve acceptable fluorescence levels. The staining reagents for biomolecules according to the present invention are highly robust and tolerant of various protein chemistries.

### [EXAMPLES]

### Example 1: Reaction time and its impact on the relative fluorescence intensity

Reaction time assessment showed in the worst cases only a 20% reduction of signal intensity when heating time is reduced to 5 min (detailed data in Table 1).

**Table 1: Time dependence of fluorescence intensity (mean values for 3 different proteins; β-galactosidase, 120 kDa; bovine serum albumin, 66 kDa; ovalbumin, 45 kDa)**

| **Reaction time [min]** | 1 | 2 | 3 | 4 | 5 | 7.5 | 10 |
|---|---|---|---|---|---|---|---|
| **Relative intensity [%]** | 66 | 74 | 87 | 78 | 90 | 108 | 100 |

### Example 2: Various compositions for the staining reagent prior to mixing with a protein sample

### Example 2A:

1 mM of the staining reagent according to formula 4 (CJ-438)
100 mM sodium phosphate buffer pH 7.5
2% SDS
10% glycerol

**Table 2: Preparation of 10 ml loading stain:**

| **Substance** | **Concentration of stock solution** | **Solvent for stock solution** | **Volume [ml]** |
|---|---|---|---|
| **staining reagent according to formula 4** | 10 mM | Acetone | 1 |
| Sodium phosphate buffer (pH 7.5) | 250 mM | Dist. water | 4 |
| SDS | 10% | Dist. water | 2 |
| Glycerol | 87% | | 1.15 |
| Dist. water | | | 1.85 |

All solutions are combined and mixed using a vortexer and aliquoted (160 µl, sufficient for one gel with 10 or 15 wells or a total sample volume of 160 µl) prior to storage at - 20 °C.

This composition, has analogously been applied to CJ-462, CJ-479 and CJ-486 given hereinbelow to produce stains with different colour but comparable sensitivity.

### Example 2B:

2 mM of the staining reagent according to formula 4
200 mM sodium phosphate buffer pH 7.5
4% SDS
20% glycerol

**Table 3: Preparation of 10 ml of loading stain:**

| **Substance** | **Concentration of stock solution** | **Solvent for stock solution** | **Amount** |
|---|---|---|---|
| **staining reagent according to formula 4** | - | | 8.2 mg |
| Sodium phosphate buffer (pH 7.5) | 250 mM | Dist. water | 7.7 ml |
| SDS | | | 400 mg |
| Glycerol | 87% | | 2.3 ml |

All solids are combined and suspended in the buffer and glycerol and dissolved by sonication for 15 min.

### Example 2C:

1 mM of the staining reagent according to formula 4
100 mM Tris-HCl pH 8.8
2% SDS
10% glycerol

**Table 4: Preparation of 10 ml of loading stain:**

| **Substance** | **Concentration of stock solution** | **Solvent for stock solution** | **Volume [ml]** |
|---|---|---|---|
| **staining reagent according to formula 4** | 10 mM | Methanol | 1 |
| Tris-HCl (pH 8.8) | 500 mM | Dist. water | 2 |
| SDS | 10% | Dist. water | 2 |
| Glycerol | 87% | | 1.15 |
| Dist. water | | | 3.85 |

All solutions are combined and mixed using a vortexer and aliquoted (160 µl, sufficient for one gel with 10 wells).

Where needed or desired, DTT can be added to the protein solution that was previously treated with the reactive stains. The tolerance of the procedure to DTT, added after reaction of the stain with the protein solution, was confirmed up to a concentration of 200 mM DTT. Upon addition of DTT, the solution was heated a second time 95 °C for 5 min. Addition of DTT effectively quenches the reactive group of the stain, which can be necessary if the tagged protein is to be purified later by chromatographic methods, to prevent irreversible covalent binding of unreacted stain to chromatographic materials.

It could be suspected that a reaction occurs between the polyacrylamide gel and the residual stain which would decrease signal-to-noise-ratios by creating a slightly fluorescent background. It was confirmed that the backgrounds of quenched (by means of DTT) samples and unquenched samples are identical, which indicates that the protein stain is not reactive towards the constituents of the gel.

### Example 3: Staining protocol according to the present invention and result thereof

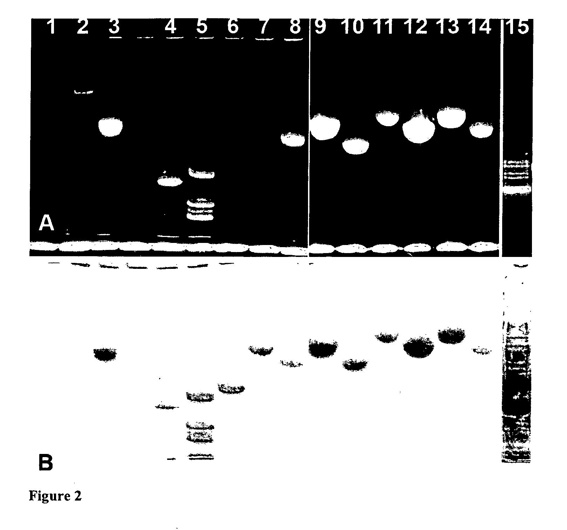

A set of proteins with different masses and cysteine content was screened for reactivity towards the staining reagent according to formula 4, see Figure 2. The samples were diluted in a modified so-called Lämmli buffer (100 mM Tris-HCl pH 8.8, 2% SDS, 10% glycerol) and the staining reagent was added to a concentration of 0.12 mM followed by denaturation and reaction (95 °C/ 10 min). Afterwards, DTT was added to a concentration of 0.2 M, followed by a second heating period (95 °C/ 10 min), to quench excessive staining reagent and reduce disulfide bridges. The test set consisted of human methionine aminopeptidase type 2 (lane 3), *E. coli* methionine aminopeptidase (4, containing impurities), West Nile virus protease (5, containing impurities), dengue virus protease (6), bovine serum albumine (7), albumine from chicken eggwhite (8) and the murein biosynthesis enzymes (MurA to MurF) from *E. coli* (lanes 3-8). One sample without protein but with the staining components was included to identify artifacts and fluorescent background associated with the stain (lane 1). Lane 2 was charged with a non pre-stained marker (peqlab protein MW) and lane 15 contains the supernatant of a cell-lysate (*E*. *coli* BL21(DE3) with overexpressed *E. coli* methionine aminopeptidase).

### Example 4: Sensitivity and linearity

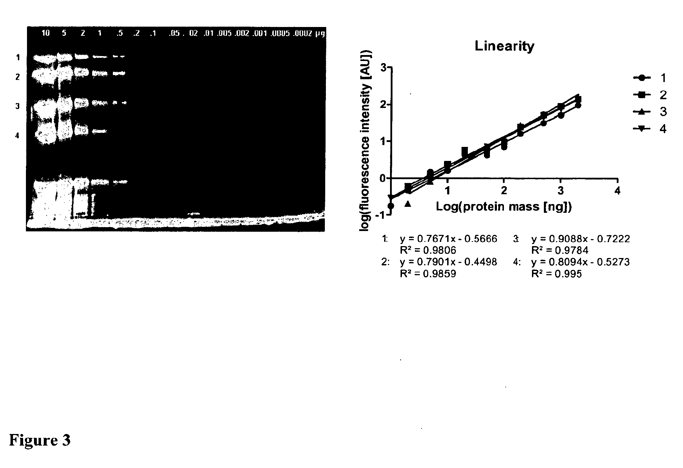

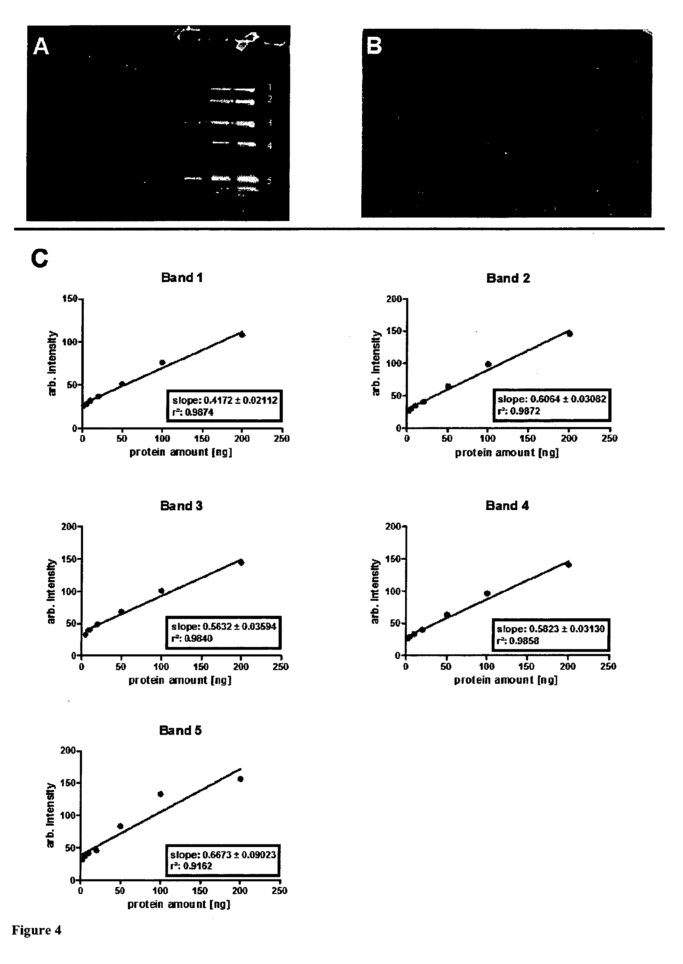

The sensitivity and linearity were assessed with suitable dilutions of a protein marker (Peqlab Protein MW, containing 100 ng/µl per band) to cover a range of 1-200 ng per band and by dilutions of the same protein marker covering a range from 0.2 ng to 10 µg (Figures 3 and 4).

The direct comparison between the silver stain and the staining reagent according to formula 4 (CJ-438) showed essentially identical but more homogenous sensitivity for the staining reagent according to formula 4. The sensitivity of the silver stain varies largely with the protein type, for some examples by more than an order of magnitude. There is no comparable effect with the staining reagent according to formula 4 (cf. Figure 4, the slopes of the linear fits are very similar for different proteins and all have high correlation coefficients). The detection limit of proteins stained with the staining reagent according to formula 4 was 1 ng and the dynamic range of at least 5000 with correlation coefficients of∼90%. A wider dynamic range could not be measured due to limitations of the used imager (12 bit camera / 4096 greyscales. When only values within the dynamic range of the camera are used, the correlation coefficients rise to >97% (cf. Figure 3; 1: β-galactosidase (120 kDa), 2: bovine serum albumin (66 kDa), 3: ovalbumin (45 kDa), 4: lactate dehydrogenase from porcine muscle (35 kDa).

In an attempt to clarify whether the high promiscuity/ unspecific binding potential of the bromoacetylbromide moiety was due to the drastic conditions used for the staining (95 °C/10 min) and whether it can be replaced by other known electrophiles, we also tested other reactive goups. Therefore, the below maleimides and hydroxysuccinimide esters were used for comparison.

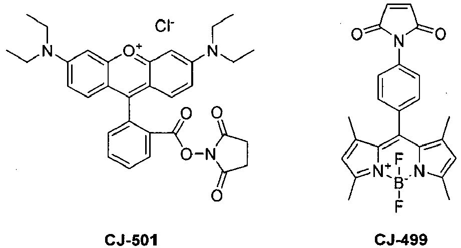

Maleimides are known to covalently attach to cysteines whereas hydroxysuccinimide esters are frequently used as lysine-reactive electrophiles. Compounds CJ-499 and CJ-501 were synthesisyed by known protocols. Loading stains were prepared in an analogous fashion to CJ-438 given hereinbelow and SDS-PAGE staining performances compared to the CJ-486 given hereinbelow and CJ-438 stain respectively. The maleimide CJ-499 gives overall a far weaker fluorescence intensity compared to the bromoacetylamide CJ-486 and has only a very minor stain intensity on the cysteine-free dengue virus protease. Therefore, the maleimide derivatives are not suitable as universal protein stains but may be used as cysteine-selective protein markers. The hydroxysuccinimide ester CJ-501 is only weakly reactive towards the tested proteins under the given conditions, despite the high abundance of lysine residues in proteins, including the tested dengue protease. CJ-501 also leads to defocussing of bands and has a non neglectable electrophoretic influence. This reactive moiety is therefore very inferior to the bromoacetylbromide. There is still a protein band visible at 15 ng of dengue protease when staining with CJ-438, demonstrating that the bromoacetylamide stain has a very low detection limit even for cysteine-free proteins. Since a significant number of proteins do not contain cysteine residues, it is very important that a covalent stain also reacts with non-cysteine amino acids.

### Example 5: Specificity and compatibility

To determine which protein residues are modified by the stain under the given conditions, model substrates were chosen and reacted with the stain. The model substrates were *n*-butanol, aminohexanoic acid methylester, imidazole, guanidine, phenylacetic acid, phenol, indole, dithiothreitol and acetamide in order to mimic the typical amino acid side chains. 40 µg of each substance in 4 µl water were combined with 16 µl of the composition according to Example 2A and heated to 95 °C for 10 min. Adducts were analyzed by ESI-MS and the intensities of the mass signal for each adduct were considered as a semi-quantitative measures of conversion. The results are given in Table 5.

**Table 5: Relative reactivity of different nucleophiles**

| **Substance** | **Corresponding AA** | **Abs. intensity** |
|---|---|---|
| Dithiothreitol | Cys | 95000 |
| Imidazole | His | 24500 |
| Aminohexanoic acid methyl ester | Lys | 11000 |
| Phenylacetic acid | Asp, Glu | 4000 |
| n-Butanol | Ser, Thr | - |
| Guanidine | Arg | - |
| Phenol | Tyr | - |
| Indole | Trp | - |
| Acetamide | Asn, Gin, backbone | - |

The stain is mainly reactive towards cysteines but also histidines, lysines and to a minor extent aspartate and glutamate, which explains why cysteine-free enzymes are also tagged by the staining reagent according to formula 4 under the given conditions.

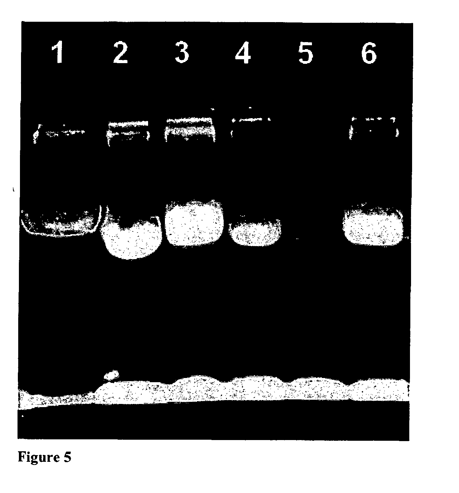

The compatibility of the staining reagent according to formula 4 with regularly used buffer ingredients was tested with samples that were readily available or that were specifically created for this test. Some were derived from other substances. First of all, the components of the loading stain are all inert towards the staining reagent according to formula 4, which means that alcohols up to 1.5 M and sulfonic acids of up to ∼0.1 mM do not interfere with the stain. This is in accordance with electrospray ionization mass spectrometry (ESI-MS adduct) formation experiments (cf. table 6), which did not show mass spectrometric signals for adducts between sulfonic acids and the staining reagent according to formula 4; even at concentrations of 1 M. An earlier version of the loading stain consisted of a Tris-HCl buffer at 100 mM, so primary amines are also tolerated in that range. Even at 1 M Tris, the stain intensity is only slightly decreased. Presence of 50 mM DTT leads to a significant decrease of stain intensity but proteins are still stained. Assuming that other typical buffer ingredients (including detergents) consist of tertiary amines, sulfonic acids and alcohol moieties, it is reasonable to expect that all typical buffer constituents are compatible to the fluorescent reactive stain. Chaotropes at typical concentrations are also well tolerated without decrease of staining intensity. Detailed data are provided in Table 6 and Figure 5.

**Table 6: Interference check for the staining reagent according to formula 4 with typical buffer constituents.**

| Substance | Functionalities | Concentration | ESI-MS | staining interference |
|---|---|---|---|---|
| **Tris** | primary amine | 1 M | adduct | lowered int. |
| | | 0.1 M | adduct | none |
| **MOPS** | sulfonic acid, tertiary amine | 1 M | - | none |
| **MES** | sulfonic acid, tertiary amine | 1 M | - | none |
| **HEPES** | sulfonic acid, alcohol, tertiary amine | 1 M | - | none |
| **ammonia** | amine | 1 M | adduct | none |
| **acetic acid** | carboxylic acid | 1 M | decomp | none |
| **urea** | urea | 8 M | adduct | none |
| **thiourea** | thiourea | 2 M | - | none |
| **guanidine** | guanidine | 8 M | - | none |
| **DTT** | thiol | 50 mM | adduct | lowered int. |
| **glycerol** | alcohol | ∼ 1.5M | n.d. | none |
| **SDS** | sulfonic acid, detergent | ∼ 0.1 mM | n.d. | none |

If necessary, the gels containing stained proteins can additionally be stained with Coomassie or silver stain after electrophoretic separation. No influence of the staining reagent according to formula 4 on the stain intensity of those methods was observed.

### Example 6: Stability of the staining reagent according to formula 4 and its compositions

The fluorescence intensity of the protein spots in gels stained with the staining reagent according to formula 4 that were fixed with formaldehyde and subsequently stored in the dark over extended periods (up to 4 weeks) remains constant. In contrast, gels that were exposed to UV irradiation for a period of 3 days on the UV table showed significant bleaching of the fluorescent dye and the sensitivity was almost entirely lost (∼1 µg detection limit, compared to 1 ng detection limit before bleaching). However, no detectable bleaching occurs on a typical timescale needed for image acquisition.

If the sample is forgotten during the staining/denaturing step for prolonged times (up to 2 h at 95 °C, loading stain according to Example 2A) the bands are slightly defocused but 50% more intense and evaluation of the gel is still possible but not quantitatively.

Three different formulations of the dye (according to examples 2A to 2C) were produced and tested for their long term stability by storing them for suitable times at different temperatures in plastic vials. The loading stain was initially stored at -80 °C to ensure its stability. However, this storage temperature is not very practical due to the fact that not every laboratory has a suitable freezer and that freezer space is often limited. The chosen temperatures for stability experiments were: room temperature (ambient lighting, ∼25 °C), fridge (dark, 7 °C) and a freezer (dark, -20 °C). After certain periods the samples were analyzed by HPLC (UV and fluorescence detection). The loading stain samples were also used to prepare SDS-PAGE samples and stain intensities were compared, the deviation of these values can be explained by the fact that only single measurements were performed and that some amount of the sample is always lost when transferring the latter into the gel pockets.

**Table 7: Stability measurements of the various formulations. For compositions of the formulations, it is referred to the "development of the stain" section. rt: room temperature, fr: fridge temperature (7 °C), -20: -20 °C.**

| **Formulation** | **Conditions** | **loss of staining reagent according to formula 4, by HPLC [%] after** | | | **loss of fluorescence intensity [%] after** | | |
|---|---|---|---|---|---|---|---|
| | | **3 days** | **1 week** | **4 weeks** | **3 days** | **1 week** | **4 weeks** |
| Example 2B | Rt | 19 | 12 | 28 | 35 | 31 | 0 |
| | Fr | 0 | 0 | 3 | 0 | 31 | 0 |
| | -20 | 0 | 0 | 0 | 0 | 16 | 9 |
| Example 2A | Rt | 0 | 0 | 29 | 34 | 0 | 15 |
| | Fr | 0 | 0 | 16 | 0 | 0 | 3 |
| | -20 | 0 | 0 | 0 | 0 | 0 | 14 |
| Example 2C | Rt | 29 | 68 | 100 | 12 | 20 | 100 |
| | Fr | 8 | 21 | 23 | 0 | 0 | 14 |
| | -20 | 0 | 0 | 0 | 0 | 0 | 0 |

The phosphate buffered formulations did not show significant decomposition even at room temperature over the period of one week. Even after 1 month only ∼30% loss of the staining reagent according to formula 4 was detected by HPLC, but no significant change in band intensity on SDS-gels was detected. The Tris buffered formulation was found to be stable at -20 °C, but decomposition occurred at higher storage temperatures and could be detected by HPLC and SDS-PAGE. After one month at room temperature the Tris stain did not contain the active ingredient in detectable amounts any more, neither did it stain proteins on an SDS-PAGE.

Due to their performance and stability, phosphate buffered formulations seem to be favorable over the Tris buffered formulation.

When individual proteins are reacted with the stain, the thus labeled proteins can be stored at 4 °C without loss of fluorescence intensity for at least 4 months. This enables staining of antibodies or other proteins and subsequent, extended storage, e.g. for blotting purposes.

### Example 7: Synthesis of the staining reagent according to formula 4 (CJ-438)

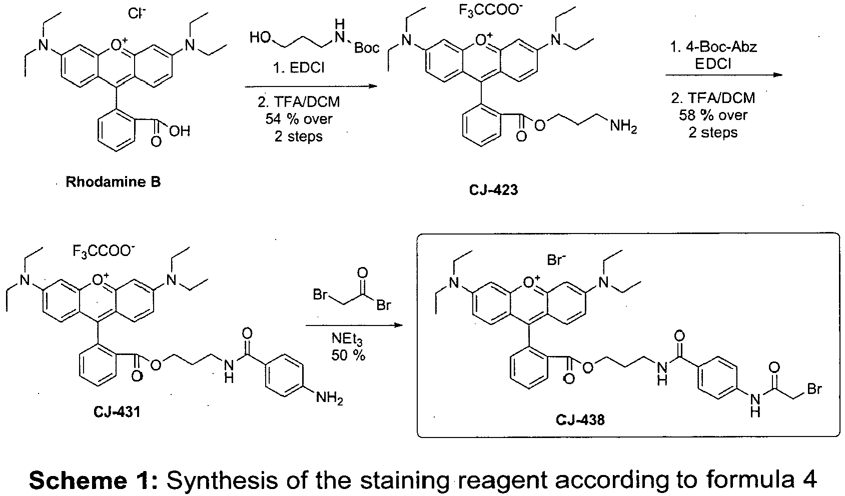

As shown in Scheme 1, the synthesis of staining reagent according to formula 4 starts with the esterification of Rhodamine B and Boc-aminopropanol using 1-ethyl-3-(3-dimethylaminopropyl)carbodiimide (EDCI) as coupling reagent. The resulting ester is then deprotected using trifluoroacetic acid to give the free amine CJ-423. The latter is then coupled to Boc-aminobenzoic acid, and the resulting amide is deprotected with trifluoroacetic acid, resulting in the compound CJ-431. As final step the electrophile is introduced with bromoacetylbromide. Normal phase chromatographic purification was only required for the isolation of CJ-423, CJ-431 and the staining reagent according to formula 4. The products of the deprotections could be obtained in pure form by extraction and washing steps.

In particular, the steps were:
Step I: 1 g of Rhodamine B (2.1 mmol), 51 mg (4-dimethylaminopyridine) DMAP (20 mol%), 440 mg EDCI (1.1 eq.) and 402 mg (1.1 eq) of *tert*-butyl 3-hydroxypropylcarbamate were dissolved in 10 ml of dichloromethane and stirred overnight in a flask wrapped in aluminium-foil. The reaction mixture was quenched with water and extracted with dichloromethane (3x). The organic layers were washed with 0.1 N HCl (2x50 ml), dried and the crude coated on silica. This was purified using MPLC (SiO₂, 50 g, dichloromethane/methanol) to obtain N-(9-(2-((3-(tert-butoxycarbonylamino)propoxy)carbonyl)phenyl)-6-(diethylamino)-3H-xanthen-3-ylidene)-N-ethyl-ethanaminium chloride as a violet solid in 50 % yield (670 mg), which was used for the following step without full characterization.
Step II: 670 mg of N-(9-(2-((3-(tert-butoxycarbonylamino)propoxy)-carbonyl)phenyl)-6-(diethylamino)-3H-xanthen-3-ylidene)-N-ethylethanaminium chloride were dissolved on 5 ml dichloromethane followed by addition of 5 ml trifluoroacetic acid. This mixture was stirred for 2 h and the solvent removed. The crude was parted between sat. Na-HCO₃ solution and ethyl acetate. The organic layer was discarded and to the aqueous layer brine and dichloromethane was added. The aqueous layer was extracted twice with dichloromethane. The combined dichloromethane phases were washed with brine (3x), dried and the solvent was removed *in vacuo.* This gave the pure product CJ-423 as a violet solid in 74% (420 mg) yield.

R*_{f}*: 10 % methanol in dichloromethane - 0.13; **¹H NMR** (300 MHz, METHANOL-*d*₄): δ 8.35 (dd, *J* = 5.7, 3.5 Hz, 1 H), 7.75 (dd, *J* = 5.7, 3.4 Hz, 2 H), 7.25 (dd, *J* = 5.8, 2.9 Hz, 7.10 (d, *J* = 9.5 Hz, 2 H), 6.94-6.82 (m, 4 H), 3.64 (q, *J* = 7.2 Hz, 8 H), 2.83 (t, *J* = 7.7 Hz, 2 H), 2.08-1.91 (m, 2 H), 1.33 (t, *J* = 7.2 Hz, 12 H), 1.21-1.09 (m, 2 H) ppm; **¹³C NMR** (75 MHz, METHANOL-*d*₄): δ 165.1, 158.6, 157.6, 155.6, 133.2, 132.7, 131.8, 131.2, 130.6, 130.3, 129.8, 114.4, 113.4, 96.5, 63.1, 46.2, 37.0, 30.8, 26.3 ppm; **HRMS** (ESI) m/z: M⁺ calcd for C₃₁H₃₈N₃O₃: 500.2908; found: 500.2900.

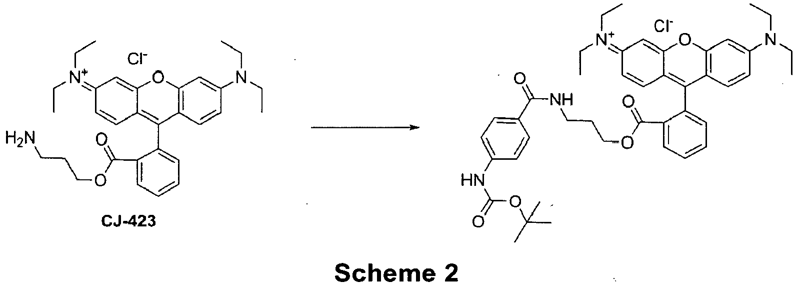

0.2 g of CJ-423 (0.4 mmol), 9 mg DMAP (20 mol%), 79 mg EDCI (1.1 eq.) and 97 mg (1.1 eq) of *tert*-butyl 3-hydroxypropylcarbamate were dissolved in 2 ml of dichloromethane and stirred overnight in a flask wrapped in aluminium-foil. The reaction mixture was coated directly on silica gel followed by MPLC (SiO₂, 25 g, dichloromethane/methanol). Thus, a violet solid was obtained in 72% yield (0.17 g).

**R*_{f}*:** 10 % methanol in dichloromethane - 0.57; **¹H NMR** (300 MHz, METHANOL-*d*₄): δ 8.35 (dd, *J* = 5.7, 3.5 Hz, 1 H), 7.75 (dd, *J* = 5.7, 3.4 Hz, 2 H), 7.25 (dd, *J* = 5.8, 2.9 Hz, 7.10 (d, *J* = 9.5 Hz, 2 H), 6.94-6.82 (m, 4 H), 4.14 (t, J = 6.1 Hz, 1 H) 3.64 (q, *J=* 7.2 Hz, 8 H), 2.83 (t, *J* = 7.7 Hz, 2 H), 2.08-1.91 (m, 2 H), 1.33 (t, *J* = 7.2 Hz, 12 H), 1.21-1.09 (m, 2 H) ppm; **¹³C NMR** (75 MHz, METHANOL-*d*₄): δ 165.1, 158.6, 157.6, 155.6, 133.2, 132.7, 131.8, 131.2, 130.6, 130.3, 129.8, 114.4, 113.4, 96.5, 63.1, 46.2, 37.0, 30.8, 26.3 ppm; **HRMS** (ESI) m/z: M⁺ calcd for C₄₃H₅₁N₄O₆: 719.3803; found: 719.3785.

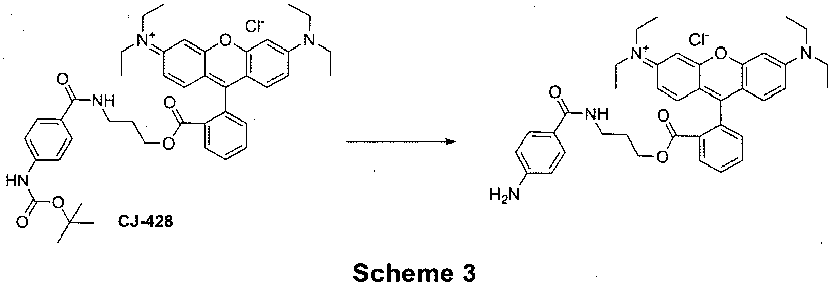

0.17 g of CJ-428 (0.2 mmol were dissolved in 2 ml of dichloromethane and 2 ml trifluoroacetic acid added followed by stirring for 2 h. The solvent was removed and the crude solved once more in dichloromethane followed by evaporation of the solvent and coating on silica. Purification by MPLC (SiO₂, 25 g, dichloromethane/methanol) yielded a violet solid in 81 % yield (0.12 g).

**R*_{f}*:** 10% methanol in dichloromethane - 0.15; **¹H NMR** (500 MHz, METHANOL-*d*₄): δ 8.33 (dd, *J* = 8.0, 1 Hz, 1 H), 7.85 (td, *J* = 7.6, 1.3 Hz, 1 H), 7.77 (td, *J* = 7.6, 1.3 Hz, 1 H), 7.54 (d, *J* = 8.8 Hz, 2 H), 7.41 (dd, *J* = 7.6, 1.3 Hz, 1 H), 7.10 (d, *J* = 9.5 Hz, 2 H), 6.98 (dd, *J* = 9.5, 2.5 Hz, 2 H), 6.93 (d, *J* = 2.5 Hz, 2 H), 6.69 (d, *J* = 8.8 Hz, 2 H), 4.10 (t, *J* = 6.4 Hz, 2 H), 3.62 (q, *J* = 7.1 Hz, 8 H), 3.18 (t, *J* = 6.4 Hz, 2 H), 1.69 (quin, *J =* 6.4 Hz, 2 H), 1.27 (t, *J =* 7.1 Hz, 12 H) ppm; **¹³C NMR** (125 MHz, METHANOL-*d*₄): δ 169.9, 166.8, 160.2, 159.3, 157.1, 135.0, 134.1, 132.5, 132.4, 131.7, 131.6, 131.6, 130.0, 115.6, 115.5, 114.8, 97.4, 64.7, 46.9, 37.7, 29.7, 12.9 ppm; **HRMS** (ESI) m/z: M⁺ calcd for C₃₈H₄₃N₄O₄: 619.3279; found: 619.3283.

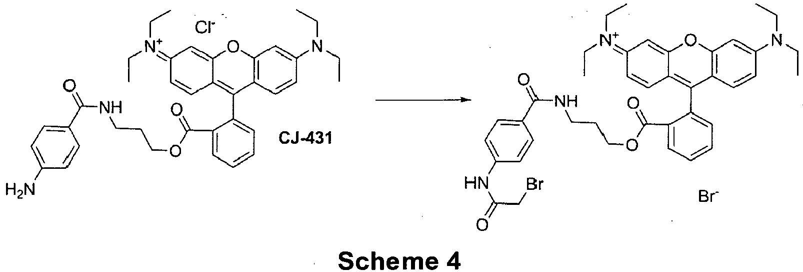

120 mg (0.1 mmol) of CJ-431, 60 µL (3 eq.) triethylamine and 12.4 µl (1 eq.) bromoacetylbromide were dissolved in 1 ml chloroform, the flask wrapped in aluminium foil and the mixture stirred over night. The reaction mixture was coated on silica followed by purification via MPLC (SiO₂, 25 g). Thus, 56 mg of the desired dark purple product, which is the staining reagent according to formula 4, were obtained (48%).

**R*_{f}***: 10 % methanol in dichloromethane - 0.78; **¹H NMR** (500 MHz, METHANOL-*d*₄): δ = 10.5 (s, 1 H), 8.31 (dd, *J* = 8.0 Hz, 1 H), 7.86-7.75 (m, 3 H), 7.71 (td, *J* = 7.7, 1.3 Hz), 7.60 (d, *J* = 8.8 Hz, 2 H), 7.01 (d, *J* = 9.29, 2 H), 6.83-6.69 (m, 4 H), 5.19 (s, 1 H), 4.25 (t, *J* = 5.9 Hz, 2 H), 4.22 (s, 1 H), 3.69-3.45 (m, 8 H), 3.29 (d, *J* = 6.2 Hz, 2 H), 1.83 (t, *J* = 6.2 Hz, 2 H), 1.36-1.29 (m, 1 H), 1.29-1.17 (m, 12 H) ppm; **¹³C NMR** (125 MHz, METHANOL-*d*₄): δ = 181.2, 166.5, 165.3, 158.7, 157.6, 155.4, 141.5, 133.8, 133.1, 131.4, 131.0, 130.5, 130.0, 129.8, 129.1, 127.7, 119.4, 114.1 113.4, 96.6, 63.2, 46.1, 36.0, 30.5, 28.5, 12.6 ppm; **HRMS** (ESI) m/z: M⁺ calcd for C₄₀H₄₄BrN₄O₅: 739.2490; found: 739.2457.

### Example 8: Synthesis of the staining reagent according to formula 5 (CJ-462)

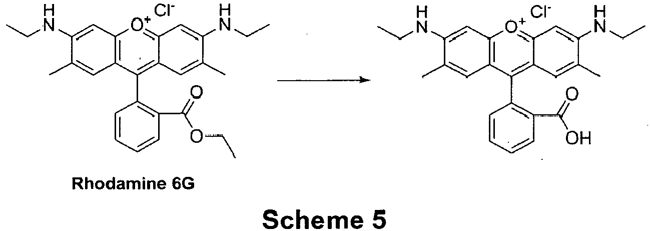

3 g of Rhodamine 6G (6.3 mmol) was dissolved in DMSO (60 ml) and 1 M NaOH was added (2 eq., 12.5 ml), followed by stirring overnight. The reaction mixture was neutralized with 1 M HCl and the precipitate collected by filtration. The solid was dissolved in 80 ml methanol and precipitated by addition of 200 ml 1 M HCl. The methanol was evaporated *in vacuo* followed by filtration and drying of the product in an exsiccator. Thus the product was obtained in 84 % yield (2.37 g).

**¹H NMR** (300 MHz, METHANOL-*d*₄) δ 8.44 - 8.24 (m, 1 H), 7.94 - 7.73 (m, 2 H), 7.47 - 7.31 (m, 1 H), 6.92 (s, 2 H), 6.89 (d, *J* = 1.03 Hz, 2 H), 3.53 (q, *J* = 7.19 Hz, 4 H), 2.14 (d, *J* = 0.88 Hz, 7 H), 1.37 (t, *J* = 7.19 Hz, 6 H) ppm.

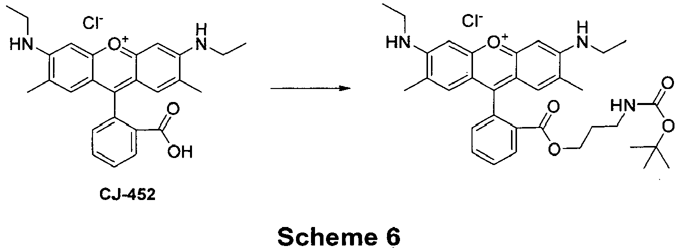

1.1 g of Rhodamine 6G acid (2.4 mmol), 60 mg DMAP (20 mol%), 940 mg EDCI (2 eq.), 2.1 ml N,N-diisopropylethylamine (DIPEA, 5 eq.) and 850 mg (2 eq) of *tert*-butyl 3-hydroxypropylcarbamate were dissolved in 10 ml of dichloromethane and stirred overnight in a flask wrapped in aluminium-foil. The reaction mixture was quenched with NaHCO₃-sol. and extracted with dichloromethane (3x) dried and the crude coated on silica. This was purified using MPLC (100 g, SiO₂) to obtain N-(9-(2-((3-((tert-butoxycarbonyl)amino)propoxy)carbonyl)phenyl)-6-(ethylamino)-2,7-dimethyl-3H-xanthen-3-ylidene)ethanaminium chloride (CJ-453) as a glassy red solid in 34 % yield (0.67 g) along with -60 % of recovered starting material.

**R*_{f}*:** 10% methanol in dichloromethane - 0.58; **¹H NMR** (300 MHz, METHANOL-*d*₄) δ 8.38 - 8.29 (m, 1 H), 7.93 - 7.76 (m, 4 H), 7.44 - 7.38 (m, 1 H), 6.94 (s, 2 H), 6.87 (dd, *J =* 6.09, 1.10 Hz, 2 H), 3.99 (t, *J =* 6.24 Hz, 2 H), 3.54 (q, *J*=7.19 Hz, 6 H), 3.12 (t, *J* = 6.83 Hz, 2 H), 2.07 - 2.17 (m, 6 H), 1.67 (qd, *J* = 6.61, 6.46 Hz, 2 H), 1.37 - 1.40 (m, 15 H) ppm; **¹³C NMR** (75 MHz, METHANOL-*d*₄) δ 159.1, 157.9, 135.6, 135.3, 134.3, 132.5, 131.7, 131.6, 131.6, 130.2, 130.1, 127.1, 127.0, 114.9, 95.0, 64.3, 60.6, 55.0, 39.6, 29.0, 28.9, 17.7, 14.2 ppm; **HRMS** (ESI) m/z: M⁺ calculated for C₃₄H₄₂N₃O₅: 572.3119; found: 572.3123.

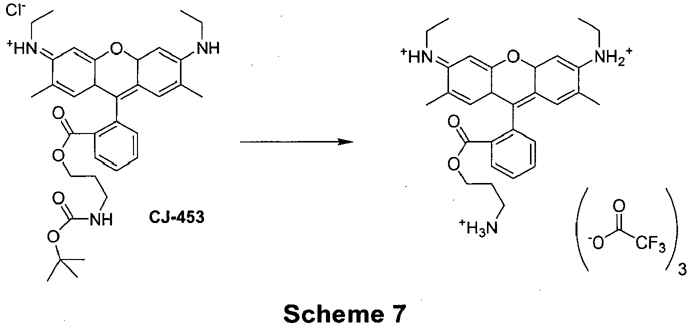

1.3 g of CJ-453 (2.1 mmol), was dissolved in 10 ml dichloromethane and 10 ml trifluoroacetic acid were added dropwise followed by stirring for 2 h. The reaction mixture was concentrated *in vacuo.* The crude was resuspended in dichloromethane and evaporated 5 times to coevaporate the trifluoroacetic acid. Thus 9-(2-((3-ammoniopropoxy)carbonyl)phenyl)-N-ethyl-3-(ethyliminio)-2,7-dimethyl-3H-xanthen-6-aminium 2,2,2-trifluoroacetate (CJ-457) was obtained as a glassy red solid in quant. yield (1.8 g).

**R*_{f}*:** 10% methanol in dichloromethane - 0.38; **¹H NMR** (300 MHz, METHANOL-*d*₄) δ 8.39 - 8.29 (m, 1 H), 7.90 - 7.77 (m, 2 H), 7.45 - 7.38 (m, 1 H), 6.93 (d, *J* = 2.94 Hz, 2 H), 6.86 (d, *J*=1.03 Hz, 2 H), 4.11 (t, *J* = 6.31 Hz, 2 H), 3.59 - 3.47 (m, 4 H), 3.14 - 2.96 (m, 2 H), 2.13 (s, 6 H), 1.96 - 1.78 (m, 2 H), 1.37 (t, *J =* 7.19 Hz, 6 H) ppm; **¹³C NMR** (75 MHz, METHANOL-*d*₄) δ 166.4, 159.1, 157.9, 135.7, 134.5, 134.3, 132.5, 131.8, 131.6, 131.5, 131.1, 130.0, 127.0, 114.9, 68.3, 39.6, 37.9, 30.9, 17.7, 13.2 ppm; **HRMS** (ESI) m/z: M⁺ calcd for C₂₉H₃₄N₃O₃: 472.2595;found: 472.2608.

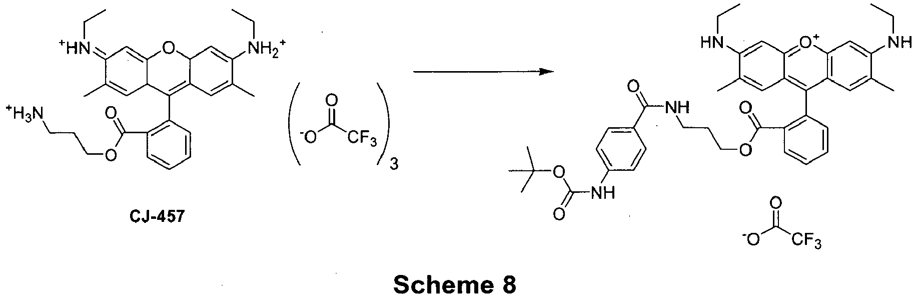

1.75 g (2.2 mmol) of CJ-456, 53 mg of DMAP (20 mol%), 825 mg EDCI (2. eq.), 1.8 ml of triethylamine (6 eq.) and 1.02 g of 4-((tert-butoxycarbonyl)amino)benzoic acid (2 eq.) were dissolved in 50 ml dichloromethane and stirred overnight. The reaction mixture was coated on silica gel and submitted to MPLC (50 g, SiO₂) to obtain 1.42 g (82% yield) of a glassy red solid still containing some triethylamine salt. To obtain an analytically pure sample an aliquot of the substance was purified via reversed phase (RP) HPLC.

**R*_{f}*:** 10% methanol in dichloromethane - 0.57; **¹H NMR** (300 MHz, METHANOL-*d*₄) δ 8.33 (dt, *J* = 7.8, 1.7 Hz, 1 H), 7.97 - 7.33 (m, 7 H), 6.99 - 6.76 (m, 4 H), 4.07 (t, *J* = 6.2 Hz, 2 H), 3.42 - 3.68 (m, 4 H), 3.13 (t, *J* = 6.8 Hz, 2 H) 2.06 - 2.17 (m, 6 H), 1.65 (dq, *J* = 6.8, 6.6 Hz, 2 H), 1.49 - 1.58 (s, 9 H), 1.25 - 1.45 (m, 6 H) ppm; **¹³C NMR** (75 MHz, METHANOL-*d*₄) δ 169.6, 167.0, 159.7, 159.5, 159.0, 157.8, 144.3, 135.6, 135.3, 134.3, 132.4, 131.8, 131.6, 130.1, 129.2, 129.1, 127.1, 127.0, 118.8, 114.9, 95.0, 81.4, 64.4, 39.6, 28.8, 17.7, 14.2 ppm; **HRMS** (ESI) m/z: M⁺ calcd for C₄₁H₄₇N₄O₆: 691.3490; found: 691.3485.

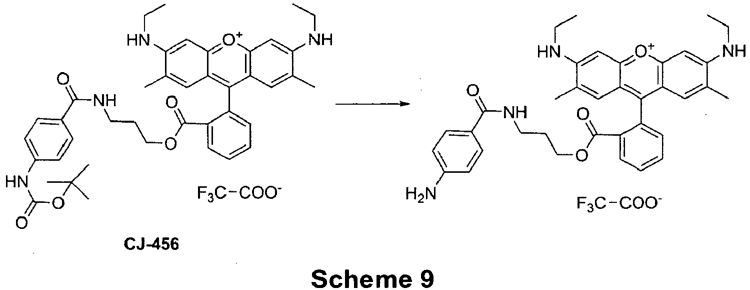

1.42 g of CJ-456 were dissolved in a 1:1 mixture of dichloromethane and trifluoroacetic acid and stirred for 2 h, then the solvent was removed *in vacuo* followed by MPLC on SiO₂ (100 g) and RP-18 (25 g, 2 runs) to obtain a glassy red solid (330 mg, 27%) alongside with 400 mg of the methylester of Rhodamine 6G.

**R*_{f}*:** 10% methanol in dichloromethane - 0.34; **¹H NMR** (300 MHz, METHANOL-*d*₄) δ 8.33 (dd, *J* = 7.85, 1.39 Hz, 1 H), 7.91 - 7.72 (m, 3 H), 7.67 - 7.57 (m, 2 H), 7.41 (dd, *J* = 7.49, 1.32 Hz, 1 H), 6.80 - 6.93 (m, 6 H), 4.06 (t, *J =* 6.17 Hz, 2 H), 3.48 (q, *J =* 7.14 Hz, 4 H), 3.12 (t, *J* = 6.83 Hz, 2 H), 2.12 (d, *J* = 0.73 Hz, 6 H), 1.64 (quin, *J* = 6.53 Hz, 2 H), 1.34 (t, *J* = 7.27 Hz, 6 H)ppm;
**¹³C NMR (75** MHz, METHANOL-*d*₄) δ 169.8, 167.0, 159.5, 159.0, 157.82, 135.3, 134.2, 132.5, 131.8, 117.5, 117.5, 114.9, 95.0, 64.6, 39.6, 37.7, 29.6, 17.7, 14.2 ppm; **HRMS** (ESI) m/z: M⁺ calcd for C₃₆H₃₉N₄O₄: 591.2966; found: 591.2965.

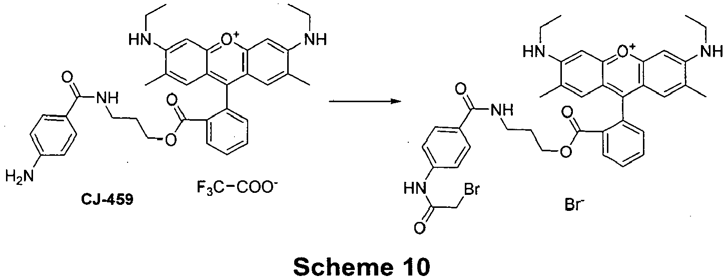

89 mg of CJ-459 were dissolved in 10 ml dichloromethane and 105 mg (6 eq.) of finely powdered K₂CO₃ was added, followed by 100 µL (9 eq.) of bromoacetyl bromide. This mixture was stirred at ambient temperature for 3 h. Then 5 ml methanol and 300 mg of K₂CO₃ were added to quench excessive bromoacteylbromide. This was stirred for 15 min and the crude product coated on silica gel followed by MPLC (25g, SiO₂) to obtain the desired product being the staining reagent according to formula 5 as a red solid in quantitative yield (100 mg).

**R*_{f}*:** 10% methanol in dichloromethane - 0.62; **¹H NMR** (300 MHz, METHANOL-*d*₄) δ 8.20 - 8.48 (m, 1 H), 7.56 - 7.98 (m, 6 H), 7.41 (dd, *J* = 7.6, 1.1 Hz, 1 H), 6.70 - 6.97 (m, 4 H), 4.08 (t, *J* = 6.2 Hz, 2 H), 4.01 (s, 2 H), 3.46 (q, *J* = 7.2 Hz, 4 H), 3.08 - 3.20 (m, 2 H), 2.11 (d, *J* = 0.9 Hz, 5 H), 1.67 (quin, *J* = 6.5 Hz, 2 H), 1.32 (t, *J* = 7.3 Hz, 6 H) ppm; **¹³C NMR** (75 MHz, METHANOL-*d*₄) δ 167.5, 166.1, 165.2, 157.6, 157.2, 156.0, 141.0, 133.5, 132.5, 130.7, 129.9, 129.9, 129.8, 129.4, 128.3, 127.5, 125.3, 118.7, 113.1, 93.27, 62.7, 37.8, 35.9, 28.1, 27.7, 16.0, 12.4 ppm; **HRMS** (ESI) m/z: M⁺ calcd for C₃₈H₄₀BrN₄O₅: 711.2177; found: 711.2163.

### Example 9: Synthesis of the staining reagent according to formula 6 (CJ-445)

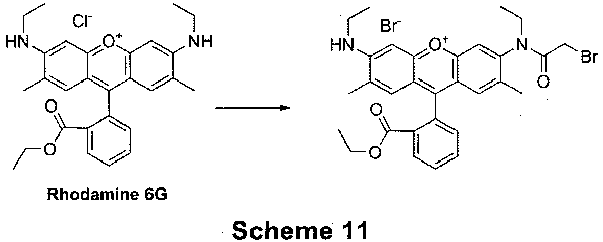

500 mg (1 mmol) of Rhodamine 6G were dissolved in 5 ml of chloroform and 91 µl (1 eq) of bromoacetyl bromide were added, followed by stirring overnight. The reaction mixture was concentrated and the crude product used for SDS-Page experiments. The ESI-MS spectrum indicated a -1:1 mixture of starting material and Product.
**MS** (ESI) m/z: C₃₀H₃₂⁷⁹BrN₂O₄⁺: 563.1533; C₃₀H₃₂⁸¹BrN₂O₄⁺: 565.1517.

### Example 10: Synthesis of the staining reagent according to formula 7 (CJ-466)

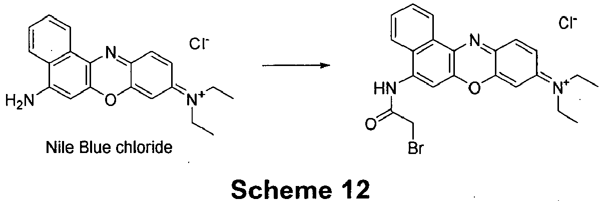

500 mg (1.4 mmol) of Nile Blue chloride (85% purity) was combined with 1 ml triethylamine (5 eq.), 35 mg DMAP (20 mol%) and 620 µl of bromoacetyl bromide in 25 ml of dichloromethane. After stirring this mixture overnight the reaction was quenched with methanol, the solvent removed *in vacuo* and the crude redissolved in dichloromethane. This solution was washed with brine (1x) and water (2x) and the solvent dried and evaporated. The crude product was successfully used for SDS-Page experiments. The ESI-MS spectrum indicated a ~1:1 mixture of starting material and Product.
MS (ESI) m/z: C₂₂H₂₃⁷⁹BrN₃O₂⁺: 438.1; C₂₂H₂₃⁸¹BrN₃O₂⁺: 440.1.

### Example 11: Synthesis of the staining reagent according to formula 8 (CJ-479)

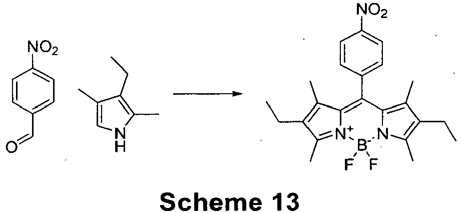

This was synthesised following the procedure provided by V. A. Azov et al. in the journal article Helvetica Chimica Acta, 2003, 86 (11), 3648-3670).
**¹H NMR** (300 MHz, CHLOROFORM-*d*) δ 8.39 (d, *J* = 8.8 Hz, 2 H), 7.55 (d, *J* = 8.81 Hz, 2 H), 2.55 (s, 6 H), 2.32 (q, *J* = 7.54 Hz, 4 H), 1.27 (s, 6 H), 0.93 - 1.04 (m, 6 H) ppm; **¹⁹F NMR** (282 MHz, CHLOROFORM-*d*) δ -145.63 (s, 2 F) ppm.

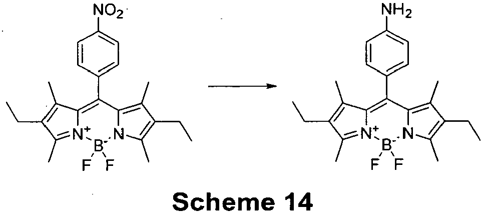

This was synthesised following the procedure provided by V. A. Azov et al. in the journal article Helvetica Chimica Acta, 2003, 86 (11), 3648-3670).
**¹H NMR** (300 MHz, CHLOROFORM-*d*) δ 7.03 (m, *J* = 8.51 Hz, 2 H), 6.80 (m, 2 H), 2.53 (s, 6 H), 2.31 (q, *J* = 7.63 Hz, 4 H), 1.40 (s, 6 H), 0.99 (t, *J* = 7.56 Hz, 6 H) ppm; **¹⁹F NMR** (282 MHz, CHLOROFORM-*d*) δ -145.86 (m, 2 F) ppm.

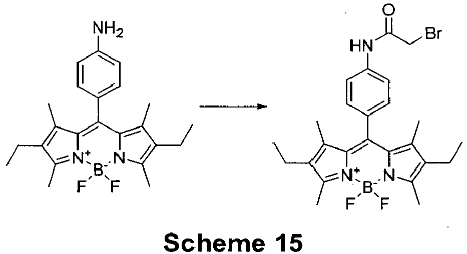

This staining reagent according to formula 8 (CJ-479) was obtained in 92 % yield (120 mg) from 100 mg starting material, following the procedure provided for CJ-462.
**¹H NMR** (500 MHz, CHLOROFORM-*d*) δ 8.26 (s, 1 H), 7.72 (m, 2 H), 7.29 (m, 2 H), 4.08 (s, 2 H) 2.54 (s, 6 H), 2.31 (q, *J*=7.58 Hz, 4 H), 1.33 (s, 6 H), 0.99 (t, *J*=7.58 Hz, 6 H) ppm; **¹³C NMR** (126 MHz, CHLOROFORM-*d*) δ 163.3, 153.9, 139.2, 138.2, 137.5, 132.8, 132.5, 130.8, 129.2, 120.0, 29.5, 17.1, 14.6, 12.5, 11.9 ppm; **¹⁹F NMR** (282 MHz, CHLOROFORM-*d*) δ -146.04 (m, 2 F) ppm; **HRMS** (ESI) m/z: M⁺ calcd for C₂₅H₂₉BBrF₂N₃O: 514.1487; found: 541.1485.

In an analogous manner, 2-bromo-N-(4-(5,5-difluoro-1,3,7,9-tetramethyl-5H-4I4,5I4-dipyrrolo[1,2-c:2', 1'-f][1,3,2]diazaborinin-10-yl)phenyl)acetamide **(CJ-486)** 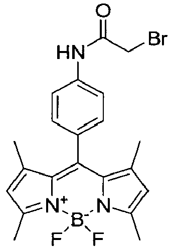 can be obtained in 59 % yield.
**¹H NMR** (500 MHz, CDCl₃) δ 8.26 (s, 1 H), 7.73 (m, 2 H), 7.30 (m, 2 H), 5.99 (s, 2 H), 4.07 (s, 2 H), 2.56 (s, 6 H), 1.43 (s, 6 H) ppm; **¹³C NMR** (126 MHz, CDCl₃) δ 163.4, 155.6, 143.0, 140.8, 137.8, 131.6, 131.5, 129.0, 121.3, 120.1, 29.4, 14.6 14.6 ppm; **¹⁹F NMR** (282 MHz, CDCl₃) δ -146.35 (m, 2 F) ppm; **HRMS** (ESI) m/z: (M+Na)⁺ calcd for C₂₁H₂₁BBrF₂N₃NaO: 482.0825; found: 482.0819.

### Example 12: Synthesis of the staining reagent according to formula 9 - guinine derivatives (CJ-491)

### (1R)-(6-methoxyquinolin-4-yl)((1R,4R,5R)-5-vinylquinuclidin-2-yl)methyl-4-((tert-butoxycarbonyl)amino)benzoate (CJ-483)

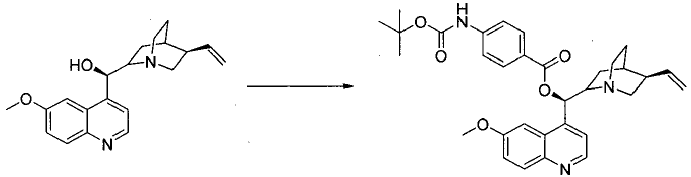

This was obtained in 38 % yield (640 mg) from 1 g of Quinine, following the procedure provided for **CJ-428.**
**¹H NMR** (500 MHz, CDCl₃) δ 8.72 (d, *J* = 4.5 Hz, 1 H), 8.07-7.98 (m, 3 H), 7.55-7.33 (m, 5 H), 6.86 (s, 1 H), 7.72 (d, *J* = 6.6 Hz, 1 H), 5.85 (ddd, *J* = 17.3, 10.2, 7.5 Hz, 1 H), 5.11-4.90 (m, 2 H), 3.98 (s, 3 H), 3.61-3.40 (m, 1 H), 3.21 (dddd, *J* = 16.3, 8.0, 5.6, 2.2 Hz, 1 H), 3.10 (dd, *J* = 13.9, 10.0 Hz, 1 H), 2.77-2.63 (m, 2 H), 2.31 (m, 1 H), 2.00-1.85 (m, 2 H), 1.85-1.65 (m, 2 H), 1.65-1.55 (m, 1 H), 1.53 (s, 9 H).ppm; **¹³C NMR** (126 MHz, CDCl₃) δ 165.1, 157.9, 152.1, 147.4, 144.8, 143.8, 143.3, 141.7, 131.8, 131.0, 126.9, 123.8, 121.8, 118.7, 117.5, 114.5, 101.5, 81.3, 74.3, 59.4, 56.8, 55.6, 42.6, 39.7, 28.2, 27.9, 27.6, 24.1 ppm; **HRMS** (ESI) m/z: (M+H)⁺ calcd for C₃₂H₃₆N₃O₅: 542.2660; found: 542.2659.

### (1R,4R,5R)-2-((R)-((4-ammoniobenzoyl)oxy)(6-methoxyquinolin-1-ium-4-yl)methyl)-5-vinylquinuclidin-1-ium 2,2,2-trifluoroacetate (CJ-488)

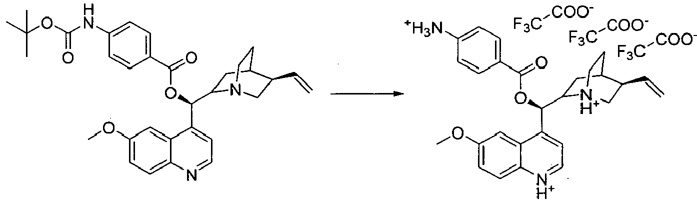

600 mg (1.1 mmol) of **CJ-483** were dissolved in 5 ml of DCM and 5 ml TFA added dropwise. This mixture was stirred for 2 h followed by evaporation of the solvent. Residual TFA was removed by coevaporation with DCM (3x). Thus the product was obtained as a glassy solid in quantitative yield (900 mg).
**¹H NMR** (500 MHz, CDCl₃) δ 8.93 (d, *J* = 5.7 Hz, 1 H), 8.38 (d, *J* = 9.4 Hz, 1 H), 7.77 - 7.93 (m, 4 H), 7.72 (dd, *J* = 9.4, 2.5 Hz, 1 H), 7.33 (s, 1 H), 6.77 - 6.62 (m, 2 H), 5.78 - 5.57 (m, 1 H), 5.19 - 5.08 (m, 2 H), 4.12 (s, 3 H), 3.94 - 3.76 (m, 1 H), 3.76 - 3.60 (m, 2 H), 3.55 - 3.36 (m, 1 H), 3.35 - 3.20 (m, 1 H), 2.83 (s., 1 H), 2.50 - 2.18 (m, 3 H), 2.18 - 1.99 (m, 1 H), 1.99 - 1.78 (m, 1 H), 1.53 - 1.23 (m, 1 H) ppm; **¹³C NMR** (126 MHz, CDCl₃) δ 163.3, 161.3, 161.2 (q, *J_{CF}* = 38.4 Hz, 3 C), 151.9, 149.9, 139.7, 136.0, 134.7, 131.5, 128.2, 127.1, 123.8, 117.7, 117.5, 117.2, 116.0, 113.8, 113.4, 100.6, 68.7, 57.6, 56.7, 54.2, 43.1, 36.1, 26.2, 24.0, 19.7 ppm; **HRMS** (ESI) m/z: (M+H)⁺ calcd for C₂₇H₂₉N₃O₃: 444.2282; found: 444.2299.

### (1 R)-(6-methoxyquinolin-4-yl)((1R,4R,5R)-5-vinylquinuclidin-2-yl)methyl 4-(2-bromo-acetamido)benzoate (CJ-491)

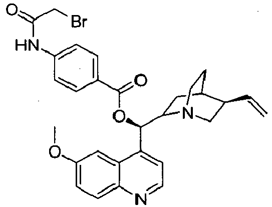

157 mg **CJ-488** were dissolved in 4 ml DCM and 280 µl triethylamine and 156 µl bromoacetylbromide were added. This mixture was stirred for 2 h followed by quenching with methanol and coating on silica gel. MPLC (SiO₂, MeOH/ethylacetate) followed by RP-MPLC afforded **CJ-491** in 51 % yield (60 mg) still containing some triethylammonium salt.

**R*_{f}*** (10 % methanol in ethylacetate): 0.38; **¹H-NMR** (300 MHz, CDCl₃): 8.87 (d, *J* = 5.3 Hz, 1 H), 8.34 (d, *J* = 9.4 Hz, 1 H), 8.00 (d, *J* = 8.7 Hz, 2 H), 7.82 (d, *J* = 2.2 Hz, 1 H), 7.58 - 7.75 (m, 3 H), 7.42 (s, 1 H), 5.50 - 5.69 (m, 1 H), 5.00 - 5.17 (m, 2 H), 4.10 (s, 3 H), 4.00 (s, 2 H), 3.50 - 3.71 (m, 4 H) 3.00 - 3.16 (m, 2 H) ppm (the missing peaks could not be assigned due to lack of purity) **HRMS** (ESI) m/z: (M+H)⁺ calcd for C₂₉H₃₀BrN₃O₄: 564.1492; found: 564.1485.

### Example 13: Production of prestained fluorescent protein markers

### Method I/II:

The DTT free protein mixture was treated with the loading stain according to the present invention. After treatment it is "ready to use". The apparent molecular weight does not change in this process. If the protein mixture contained DTT it was left in a dust free area for 3 days and evaporated solvent was replenished with water before treatment with loading stain. If different band colours are desired this method can be altered as mentioned under method III.

### Method III:

The protein (β-galactosidase from *E. coli,* bovine serum albumine, *E.coli* methionine aminopeptidase, lysozyme from chicken egg white) was dissolved in 100 mM phosphate buffer at pH 8.8 at a concentration of -20 mg/ml and mixed with the 2x loading stain of desired colour in a 1:1 ratio. This was then heated to 95 °C for 10 min and the mixture loaded on a preparative mini gel of appropriate concentration (8.3x6.0 cm (WxH), max amount of protein 5 mg in ~500 µl). The desired protein band was cut out of the gel and placed in a sealable tube. To this 1 ml elution buffer per mg protein was added and the tube placed on a rotary shaker over night to elute the protein by diffusion. The solution was decanted and if necessary concentrated by centrifuge ultrafiltration concentrators or dialysis. The individual proteins are then mixed to obtain a protein marker of the desired range and Lämmli loading buffer added in a 1:1 ratio. This is heated to 95 °C for 10 min to obtain a ready to use marker. The apparent molecular weight has to be determined in comparison with a suitable marker.

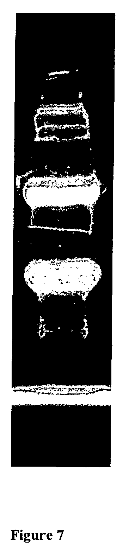

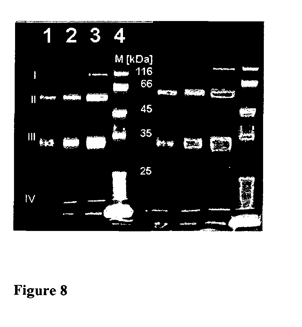

If the proteins are of sufficient quality the procedure can be altered:
After the staining reaction DTT is added to a concentration of 10 mM and the mixture is heated once more to 95 °C. The individual proteins can now be mixed to obtain the desired range of protein masses. (exemplary gel pictures are depicted in Figures 7 and 8)

### Method IV:

The protein (bovine serum albumine, *E.coli* methionine aminopeptidase) was dissolved in 100 mM phosphate buffer at pH 8.8 at a concentration of ~10 mg/ml and CJ-438 or CJ-462 added to 1 mM (from a 40 mM stock solution in methanol or acetone) and this mixture was placed in an incubator at 37 °C (or room temperature) for 2 h, to this DTT was added to a concentration of 10 mM followed by incubation for another 2 h. this mixture was purified via size exclusion chromatography. The fractions containing the desired protein were pooled and if necessary concentrated. The individual proteins are then mixed to obtain a protein marker of the desired range and Lämmli loading buffer added in a 1:1 ratio. For SDS-PAGE this is heated to 95 °C for 10 min to obtain a ready to use marker. For native PAGE, bromophenole blue is added to a concentration of 0.001 %.

## Claims

1. A staining reagent for biomolecules according to the general formula (1): 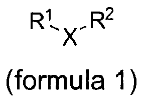 wherein
R¹ is a fluorescent dye, selected from the group consisting of Rhodamine B, Rhodamine 6G, Rhodamine 123, Sulforhodamine B, Sulforhodamine 101, Carboxy-Rhodamine, Rosamine, Texas Red, Fluorescein, Carboxyfluorescein, Cy3, Cy5, Cy7, Nile blue, quinine and the class of BODIPY dyes,
wherein R² is a reactive moiety according to the following formula (2) 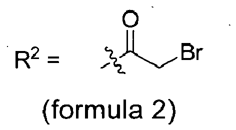 and wherein X is a spacer or X is absent, such that R¹ and R² are connected via a single bond.

2. The staining reagent for biomolecules according to claim 1, wherein, if X is a spacer, X is a spacer according to the general formula (3) 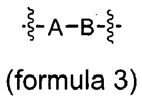 wherein
A is an aliphatic hydrocarbon with 1 to 10 carbon atoms and optionally having (i) two terminal hydroxyl groups, (ii) two terminal amino groups, (iii) two terminal methylamino groups, (iv) one terminal hydroxy group and one terminal amino group or (v) one terminal hydroxy group and one terminal methylamino group, each of the groups being at the opposite terminals of the aliphatic hydrocarbon group, and wherein A is bound to R¹ via an ester or an amide bond and wherein A is bound to B or R² via an ester or an amine bond, and wherein
B is aminobenzoic acid, which is bound to R¹ or A via an ester or an amide bond and which is bound to R² via an ester or an amide bond with the proviso that, if X is not absent, at least one of A and B is present.

3. The staining reagent for biomolecules according to claim 1 or 2 selected from any one of the following formulae (4) to (9):

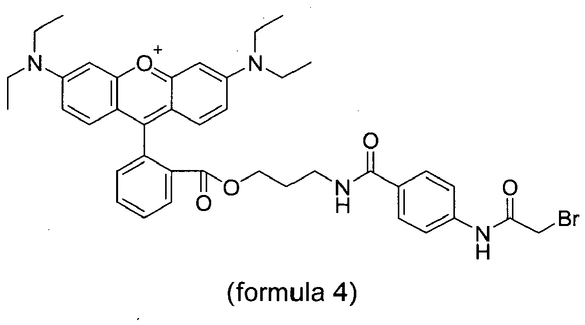

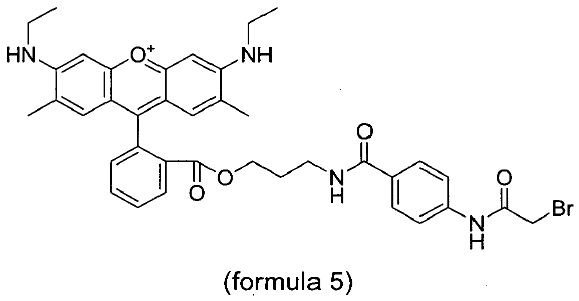

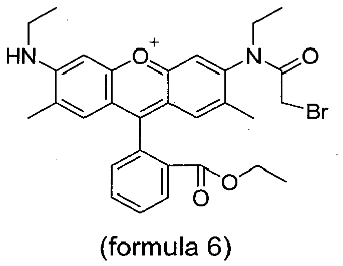

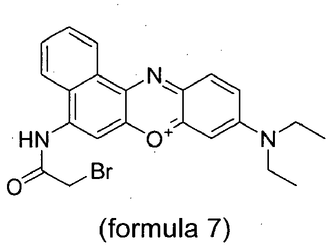

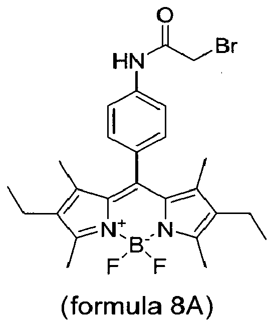

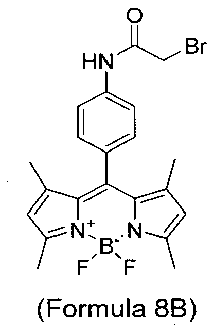

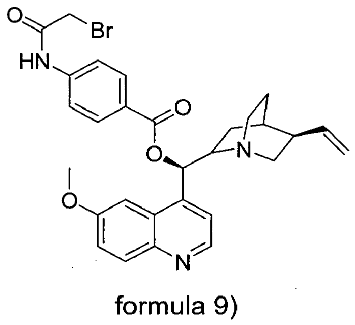

4. A method of staining a protein sample with the staining reagent for biomolecules according to any one of claims 1 to 3, wherein the staining reagent and a protein sample comprising one or more proteins are reacted and conjugates of said protein staining reagent with one or more proteins are produced.

5. The method according to claim 4, wherein a protein sample comprising one or more proteins is subjected to a protein separation procedure prior to being reacted with the staining reagent for biomolecules.

6. The method according to claim 4, wherein a protein sample comprising one or more proteins is subjected to a protein separation procedure after being reacted with the staining reagent for biomolecules.

7. The method according to claim 4 or 6, wherein the reacted protein sample is monitored during separation using a fluorescence detection method.

8. The method according to any one of claims 4 to 7, wherein the protein sample comprising one or more proteins is subjected to polyacrylamide gel electrophoresis and the gel is not washed after performing the reaction according to claim 4 and prior to fluorescent detection of the protein bands.

9. A method for producing the staining reagent for biomolecules according to any one of claims 1 to 3, wherein a fluorescent dye selected from the group consisting of Rhodamine B, Rhodamine 6G, Rhodamine 123, Sulforhodamine B, Sulforhodamine 101, Texas Red, Fluoresceine, Carboxyfluoresceine, Cy5, Cy3, Cy7, Nile blue, quinine and Bodipy is reacted with the reactive moiety either directly or via the spacer X.

10. A kit comprising a stock solution of the staining reagent for biomolecules according to any one of claims 1 to 3.

11. Use of the staining reagent for biomolecules according to any one of claims 1 to 3 for staining a protein sample comprising one or more proteins.

12. Use of the staining reagent for biomolecules according to any one of claims 1 to 3 for staining a tissue for microscopic analysis.

13. A prestained protein molecular weight marker composition, comprising at least two different proteins, said at least two proteins having differing molecular weights, wherein said at least two proteins are stained with at least one staining reagent according to any one of claims 1 to 3.

14. The prestained protein molecular weight marker composition according to claim 13, wherein each of the at least two proteins is stained with a different staining reagent.

15. A method for producing the prestained protein molecular weight marker composition according to claim 13 or claim 14, comprising the steps of
(a) providing said at least two proteins in a buffer that is free of thiols and
(b) reacting said at least two proteins with at least one staining reagent according to any one of claims 1 to 3, thereby producing conjugates of said at least one staining reagent with said at least two proteins.
